# EUROPEAN PATENT SPECIFICATION

(11) **EP 1 502 624 B1**
(45) Date of publication and mention of the grant of the patent: **14.02.2007**
(21) Application number: 04024620.9
(22) Date of filing: 14.08.2001
(51) Int. Cl.: A61N 1/375

(54) **Stimulation catheter with electrode and optical fiber**
Stimulationskatheter mit Elektrode und Faseroptik
Cathéter de stimulation avec une électrode et une fibre optique

(30) Priority: 17.08.2000 US 225823 P
(43) Date of publication of application: 02.02.2005
(62) Divisional of application: 01119555.9
(73) Proprietor: Borkan, William N., North Miami Beach, Florida 33160 (US)
(72) Inventor: Borkan, William N., North Miami Beach, Florida 33160 (US)
(74) Representative: Strehl Schübel-Hopf & Partner

(56) References cited:
- WO-A-94/16623
- US-A- 4 900 303
- US-A- 5 885 258
- US-A- 5 935 102

## Description

The present invention relates to an implanted tissue stimulator system and catheters and more specifically for use in the intrathecal space.

The concept of using electronic stimulation systems for the purpose of controlling nerves or muscles is well known. These systems typically utilize an implantable or an external pulse generator. The external systems consist of a transmitter and antenna which transmits energy and/or stimulation signals transcutaneously through a patient's skin to an implanted receiver. The receiver provides signal processing of the received pulses and transmits the energy derived therefrom to activate electrodes implanted adjacent to specific types of tissue to be stimulated. A system like the one described above has been disclosed previously in U.S. Patent 3,727,616. It is also known in prior art where more than one pair of electrodes are activated such as Patent 3,449,768.

Problems arise in these prior art systems where electrode placement fails to provide the desired physical response. It may also occur later if a change in patient condition or electrode position occurs. This failure may also be caused by improper polarity of the stimulated electrodes relative to one another. Furthermore, it is often required that the electrodes be implanted surgically adjacent to one or more nerve fibers. This type of procedure involves inherent risks due to the fact that it is often performed in close proximity to the brain or spinal cord or other sensitive nerves or tissues. It is therefore desirable to perform the electrode implantation only once to minimize the surgical risks to the patient as well as the financial burdens. Moreover, even when a plurality of electrodes have been utilized, such that repeated surgical procedures are not required, the prior art systems did not provide for dynamic programming and reprogramming of different electrodes after surgery until U.S. Patent 4,459,989 to Borkan.

The Borkan patent '989 disclosed an external stimulator system which allowed non-invasive programming of the stimulated electrodes. Each electrode was capable of assuming a positive, negative or open circuit status with respect to the other electrodes. This effectively allowed the electrodes to be "repositioned" non-invasively. That same programming ability (plus/minus/off) was later applied to totally implantable systems as well. The system had mono/biphasic control also. Further improvements are described in U.S. Patent 4,612,934 also to Borkan.

The application of spinal cord stimulation has shown itself to be effective in the treatment of pain and is under study for various other medical conditions. Initially, the leads were implanted by laminectomy and applied to the dura in the epidural space. The next generation of electrodes were positioned by percutaneous implantation. These were either placed into the intrathecal space or the epidural space. Due to the construction and nature of the electrodes used at that time (approximately 30 years ago), numerous complications occurred with the use of intrathecal catheter electrodes. These included CSF leakage. In addition, intrathecal electrodes were prone to significant movement and migration (as were the early epidural leads).

Therefore, development efforts were focused on percutaneous implantations in the epidural space. An example of a multi-electrode catheter assembly for spinal cord stimulation is shown in U.S. Patent 4,379,462 to Borkan.

Advances in catheter technology have allowed the widespread application of intrathecal catheters that deliver drugs for various medical applications. In addition, various fixation means for catheters have been developed and successfully utilized to eliminate the problem of electrode movement and migration. Therefore, it is now possible to develop a catheter electrode for placement into the intrathecal space without the problems and complications experienced previously.

The recent use of totally implantable stimulator systems with an implanted power source have resulted in increased emphasis on the amount of power required to deliver an effective stimulation regimen. In addition, use of multi-electrode systems has put an even greater strain on the limited resources of an implanted power cell.

The intrathecal space provides a more direct means of delivering either drugs or electrical stimulation to the spinal cord. By definition, implantation of devices in the epidural space place stimulation or drugs outside the dura, significantly further away from the spinal cord. Intrathecal placement therefore allows significantly reduced levels of stimulation and drugs to create the same effect as a catheter placed epidurally.

US-A-5,423,877 discloses a catheter electrode assembly which has the features included in the first part of claim 1. The device is for use in acute pain management and has a plurality of spaced electrodes and a conduit for the infusion of medications simultaneously with the application of stimulation pulses to the epidural space of the spinal cord.

US-A-4,900,903 discloses a catheter which has the features included in the first part of claim 1.

The invention defined in claim 1 permits the insertion and fixing of the catheter at a point and treating tissue along the length of the distal end. The passage may be used for delivery of drugs, thermal or photonic energy. It may also be for used a stilet during positioning of the catheter. The fibre-optic channel can provide photonic energy to the tissue as well as function as a lens for a remote camera.

Other objects, advantages and novel features of the present invention will become apparent from the following detailed description of the invention when considered in conjunction with the accompanying drawings.

### BRIEF DESCRIPTION OF THE DRAWINGS

Figure 1 is a cross-sectional view of a backbone and spinal cord.
Figure 2 is a perspective view of a catheter having 270° electrodes.
Figure 3 is a perspective view of a catheter lead with 90° electrodes positioned on the spinal cord.
Figure 3A is a perspective view of the 90° electrode.
Figure 4 is a perspective view of a catheter with a 60° electrode positioned on the spinal cord.
Figure 4A is a perspective view of the 60° electrode.
Figure 5 is a perspective view of a catheter with an additional common anode.
Figure 6 is a perspective view of another catheter with an additional common anode electrode.
Figure 7 is a perspective view of a catheter including a passage having an outlet at the tip of the electrode and a balloon fixation device. Provision for an optional stilet is also shown.
Figure 8 is a perspective view of a catheter with an outlet between the electrodes and a nitinol fixation device.
Figure 8A shows the nitinol fixation device in position prior to deployment.
Figure 9 is a perspective view of a catheter with a passage having an outlet on the electrode and a tine fixation device.
Figure 10 is a perspective view of a catheter wherein the passage is external the sheath.
Figure 11 is a perspective view of a catheter electrode with a passage as well as two optical channels according to the principles of the present invention.
Figure 12 is a nerve cuff lead employing a common anode.
Figure 13 is a cross-sectional view of another catheter with a 90° electrode of reduced area.
Figure 14 is a perspective view of a catheter electrode having a bent distal end.

### DETAILED DESCRIPTION OF THE PREFERRED EMBODIMENTS

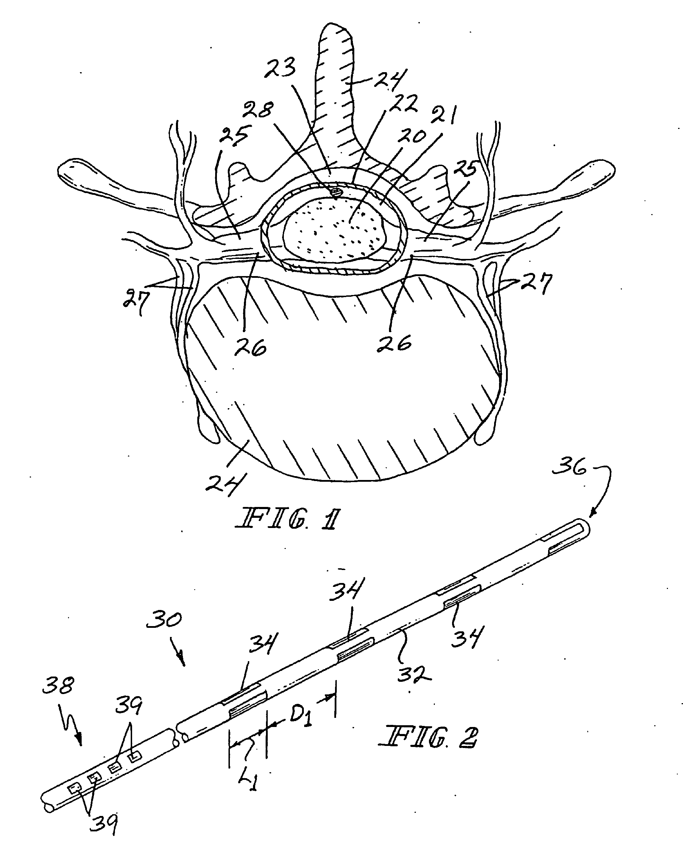

Electrodes used for spinal cord stimulation are typically implanted in the epidural space 23 between the vertebra 24 and the dura 22, shown in Figure 1. This is done for various reasons, including reduced complexity of the surgery, reduced potential complications, an increased stability of the implant. However, implantation in the epidural space requires a significant amount of additional stimulation power since the signal must be transmitted through the dura 22 and epidural space 23 and cerebrospinal fluid in order to reach its desired neural targets in the spinal cord 20. Anterior roots 25 and posterior roots 26 of the spinal cord 20 and Rami Communicantes 27 are also shown.

There were early stimulator leads 28 which were utilized in the intrathecal space 21 between the spinal cord 20 and the dura 22 without success. Typically, these leads resulted in inconsistent stimulation, overstimulation and, generally, ineffective therapy. However, they utilized technology approximately thirty years old. Due to development and implementation of better epidural leads, as well as the lack of a concern for energy requirements (since they were using an external battery pack), this methodology was quickly abandoned.

More recently, intrathecal catheters have been utilized extensively for administration of drugs including morphine and baclofen via implantable drug pumps. Catheter complications were so prevalent thirty years ago (CSF leakage, migration, etc.) are no longer a significant problem. Of course, administration of a drug does not require a precise electrode placement.

This new intrathecal neurostimulation electrode would uses for example 4 mm electrode contacts (more forgiving when it comes to precise electrode placement) and yet would require dramatically less power (less than 20% of the power required for current epidural electrodes) because the electrodes would be in direct contact with the CSF and much closer to the desired neural targets. The primary insulating factor resulting in higher power requirements for epidural leads is the lack of conductivity of the dura 22. The shunting effect of electrical current within the CSF is a much less significant factor. Therefore, an electrode designed with a stabilization means or fixing elements for use in the intrathecal space 21 utilizing materials generally accepted in the art for intrathecal drug administration would provide a unique, novel and advantageous method of stimulation of the spinal cord.

Fixation methods could include one or more of the following: inflatable balloons, tines which are either retractable with a stilet or simply pliable enough to fit through a specially designed introducer or a novel shape (D shape) or the use of nitinol. Any of these fixation methods, singly or in combination, can be used to provide stable electrode placement on a long term basis.

The advantages of an intrathecal electrode are dramatically reduced size and/or increased life of a neurostimulator implant. In addition, implantation in the intrathecal space allows the use of a larger diameter electrode catheter and, therefore, more electrodes can be placed, providing a wider selection of stimulation sites and stimulation paradigms. These stimulation paradigms could include sophisticated programs that switch stimulation between a number of electrodes slowly (over seconds or minutes, hours or days) to avoid accommodation of the stimulation or could be fast (approximately the same speed of the electrical activity of neurons in the spinal cord) artificially generating neural signals along the spinal cord 20 which could be perceived as any other sensory function with signals that travel through the spinal cord. For instance, a signal could be generated that would correspond to heat being applied to a patient's big toe, or pressure being applied to a patient's foot, or the sensation of a limb in a different orientation than it actually is.

Theoretically, tastes, smells, sights or even thoughts could be created in this manner allowing various artificial prosthesis (visual, auditory, etc.) to interface with the human body.

A first embodiment is illustrated in Figure 2. A catheter 30 includes a sheath 32 having a plurality of electrodes 34 at a distal end 36 of the catheter 30. A proximal end 38 of the catheter 30 have contacts 39 to be connected to a stimulator, not shown. There is one contact 39 for each electrode 34. The length of the electrodes L1 are generally 2 to 4 mm. The distance D1 between the electrodes is typically 6 mm, for example. The electrodes are shown to extend 270° about the circumference of the catheter.

In order to achieve further increases in battery life, the surface area of the active electrode contact can be reduced. An electrode with a specific orientation adjacent to the spinal cord would allow a 30° - 270° electrode as opposed to the 360° electrodes used in all current catheter electrodes. This would also enable the electrodes to remain at the preferred 3 or 4 mm in length making it easier for physicians to hit the desired target and requiring fewer electrode contacts in the catheter (also allowing a smaller catheter). Different means could be used to stabilize the electrode in place, including (1) inflatable balloons, (2) nitinol, (3) novel shape electrodes, (4) mechanical system whereby tines are deployed upon removal of the stilet, (5) mechanical system of flexible tines.

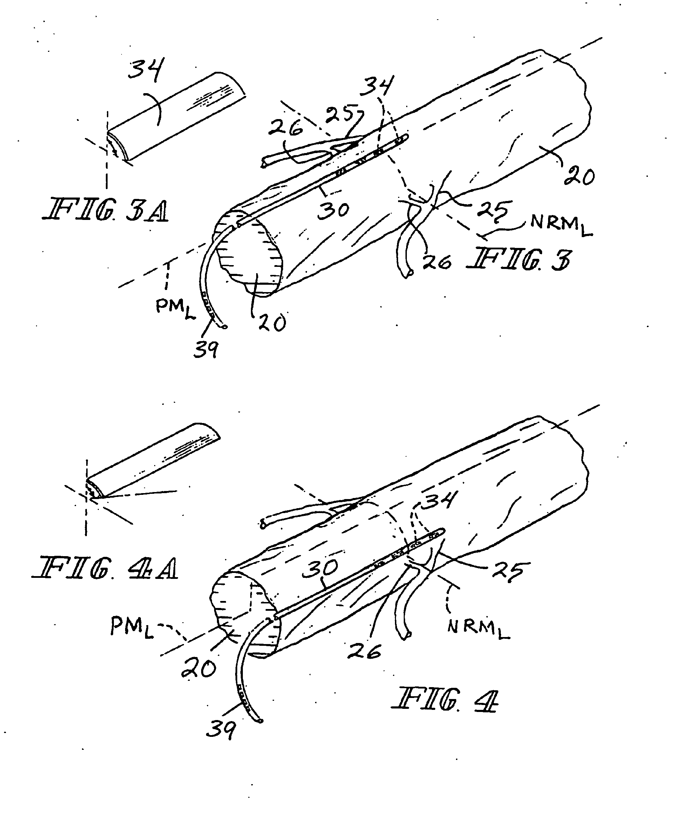

A 270° electrode is illustrated in Figure 2, a 90° electrode is illustrated in Figure 3A and a 60° electrode is illustrated in Figure 4A.

The small radius electrode, for example, 90° or less, allows a focused electrical stimulation field along the physiological midline PML of the spinal cord 20 and reduces the effective stimulation field in the area of the bending fibers. As illustrated in Figure 3, the electrode 34 may also be placed at the nerve root midline NRML It is well known that bending fibers are preferentially stimulated when compared to longitudinal fibers. Thus, by positioning the catheter adjacent to the desired longitudinal fibers, a focused stimulation of the longitudinal fibers will take place.

The small electrodes in the range of 30° - 60° also allows selective activation of the fibers. This is particularly useful where the nerve enters the cord. Such placement is illustrated in Figure 4, where the 60° electrode 34 is adjacent to the nerve roots 25, 26.

For any of the above designs, an additional method of decreasing power requirements is to use monopolar stimulation. A large surface area common anode electrode is used in conjunction with a singles electrode in the desired stimulation area on the electrode contact array. Monopolar stimulation is not new, but does provide the opportunity to decrease the power requirements for effective stimulation.

The incorporation of a large surface area anode electrode some distance away from the other active electrodes on the same catheter or along the length of an intrathecal catheter has never been performed or attempted. In the prior systems, the casing of the stimulator acted as the anode in a monopolar mode. Its distance from the distal electrodes created a long current path. This new and novel approach reduces the current path, creates a different field and also allows a unitary electrode system so that the stimulator device itself does not have to be made in two different configurations (one with an active case anode and one without). Therefore, a choice between bipolar and monopolar stimulation can be made after implantation using a stimulator (pulse generator) which could normally only provide bipolar stimulation.

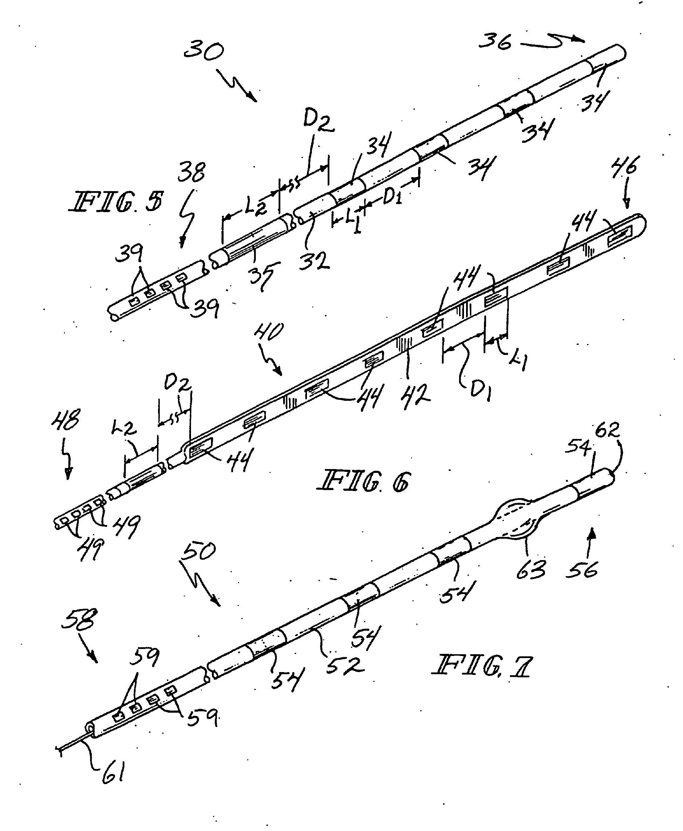

As illustrated in Figure 5, an additional electrode 35 is provided at the distal end 36 displaced from the other electrodes 34. The electrodes 34 are shown as 360° electrodes. As illustrated in Figure 6, catheter 40 includes a sheath 42 with having a plurality of inline electrodes 44 and an additional electrode 45 on the wire or sheath extension 47 extending from the distal end or paddle 46. The proximal end 48 has contacts 49 connected to each electrode and to a stimulator.

The electrodes 34, 44 all have a length L1 and the additional electrode 35, 45 has a length L2. Length L2 is greater than L1, at least twice its length. Thus, for example, if length L1 is 2 mm, the length L2 is 4 mm. The length L2 may be anywhere between 2-4 times that of the length L1. Also, it should be noted that the additional electrode 35, 45 is spaced by a distance D2 from the nearest electrode 34, 44. Where D1 is approximately 6 mm, the distance D2 is at least 10 mm and can be as much as 20 mm. With this distance, the electrode acts as a point source when used in conjunction with a second electrode. The electrodes 34, 44 act as a point source when used in conjunction with the additional electrode 35, 45 of the increased area.

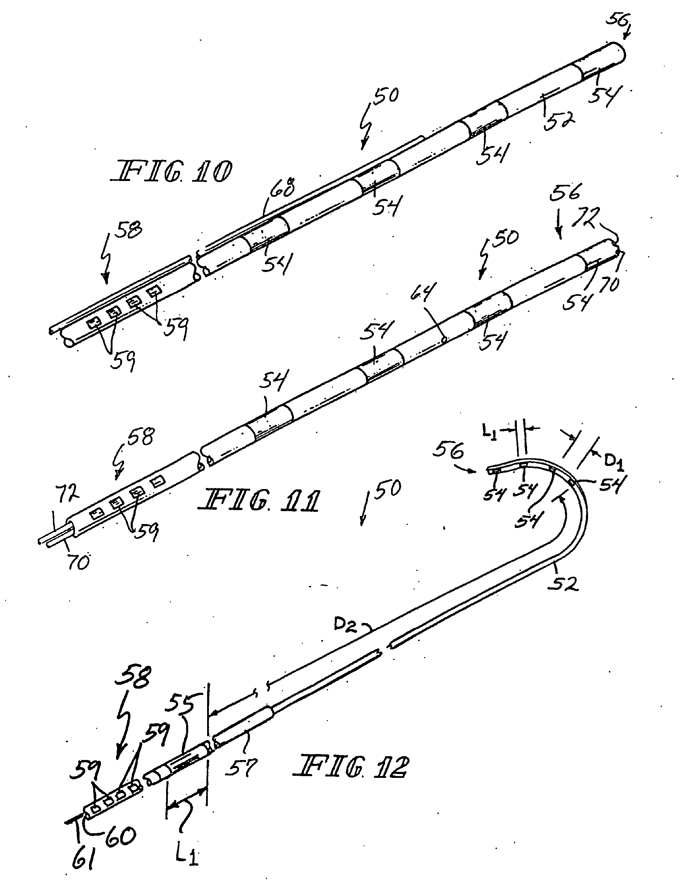

Alternatively, a common anode or additional electrode 35 may be the same length as the other electrodes 34, but have a greater circumferential dimension than that of the electrode 34. This would increase the surface area of the additional electrode 35 relative to the other electrodes 34. Therefore, the electrodes 34 may be in the 30° to 270° configuration previously described. The separation difference between the electrodes 34 and that of 35 will allow operation as described with respect to Figure 5 and 6. Such an electrode is illustrated in Figure 12 to be discussed below.

A catheter capable of stimulation electrically as well as dispensing drugs is illustrated in Figures 7-11. The catheter 50 has a sheath 52 with inline electrodes 54 spaced along the distal end 56. At the proximal end 58, terminal contacts 59 are connected internally to each of the electrodes shown. A passage 60 is provided in the sheath 52. In Figure 7, an outlet 62 is provided in the tip of the distal end 56 and a balloon fixation device 63 is shown. Provision is also made for an optional stilet which is removable and may be used to assist in placement of the catheter. Various stilets 61 of different shapes and characteristics may be used with these leads.

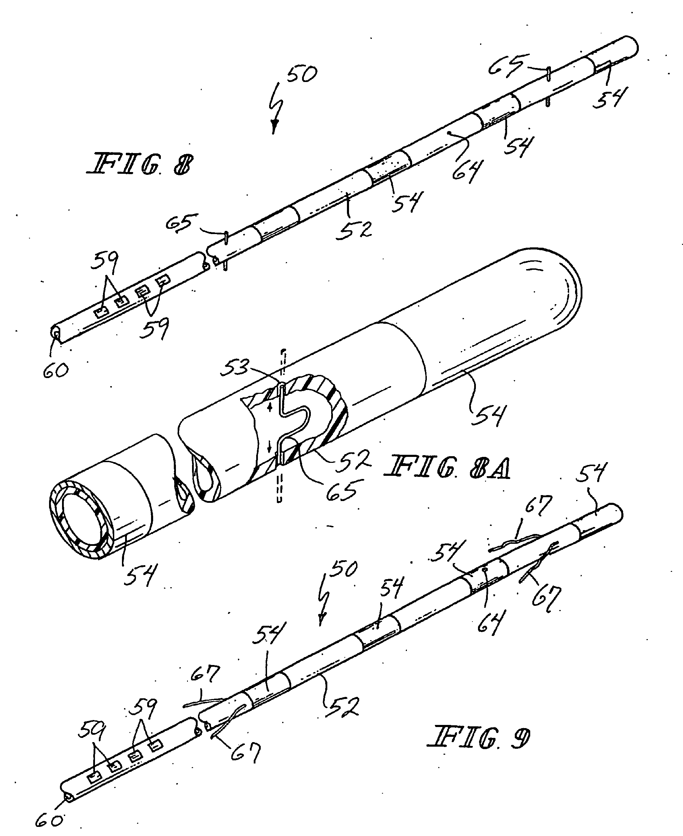

In Figure 8, the outlet 64 is shown in the space between the electrodes 54 and a nitinol fixation device 65 is shown in the deployed condition. In figure 8A, nitinol fixation device 65 is shown prior to the deployment through opening 53 in sheath 52. In Figure 9, the outlet 64 is shown in or on the electrode 54 and a tine fixation device 67 is shown.

Although one fixation is illustrated on a specific Figure, any fixation device may be used with any of the catheters. Fixation methods may also include devices that are actively deployed and/or retracted (for instance by a stilet) in addition to the methods shown herein. Also, the fixation device may be located at any or more than one location or position along the catheter. A fixation device should be used where the catheter electrode is installed in the intrathecal space.

While Figures 7-9 show the passage 60 internal to the sheath 52, an external delivery 68 may be used as shown in Figure 10.

The catheter 50 may also include a single or pair of optical channels 70 and 72 having outlets or ports in the distal end. The fiberoptic channels and light energy delivered through a clear translucent area in the catheter is illustrated in Figure 11. One of the channels can provide a source of light to be used as a further source of stimulation. The other channel will form a lens for a camera or other monitoring devices. The camera can be used in positioning the electrode or distal end. Channel 60 with outlet 64 is also shown for a drug delivery.

It should be noted that only a single optical path can be used to provide a source of light for a photon stimulation without the passage 60 or the outlet 64. It should also be noted that the passage 60 or external passage 68 for drug delivery may be used in combination with the light channel 70 without the electrodes 54. Although two channels 70, 72 are shown, any number of channels could be used and could include a combination of different types of channels - working channels for instruments, optical channels for light or camera, stilets etc.

The catheter of Figures 7-11 may be used in a percutaneous drug treatment method. The drug is administered to the patient and the stimulation cathode is positioned adjacent the tissue to be treated by the drug. The tissue is then stimulated using the catheter. This allows selective and localized drug treatment. Certain compounds change chemically when stimulated. Compounds can also be delivered via electropheretic means.

The intrathecal location of this catheter places it past the blood/brain barrier and therefore offers numerous unique opportunities for combination stimulation/drug treatment regimes. In addition, application of various forms of energy (heat, cold, etc.) independently or with stimulation allow indirect alteration of brain chemistry. Also the production of heat in a tissue may make the tissue more susceptible to absorption of a drug. The drug may be administered through the passage 60 or 68. The source of stimulation and/or the drugs may be external to the body or totally implantable. The implantable system could include a microprocessor, pump, port and an external port for refilling the pump or selection of a different drug or fluid.

The drug may be stimulated by electrical energy using the electrodes 54 or by photonic energy using the optic channel 70. Heat may be produced by either the electrodes 54 or the optical channel 70. Cold may be produced by a Peltier effect chip or other means, for example gas or liquids. If the drug is responsive to light energy of a specific wavelength, the stimulation uses light energy of that specific wavelength.

Finally, intrathecal stimulation by light energy may be administered via the catheter - with or without drugs or electrical stimulation - to activate certain tissues. These tissues may, in some cases, be treated in some manner to increase their sensitivity to this method of activation and maybe used in combination with electrical stimulation.

The catheter 50 as illustrated in Figure 12 is curved at its distal end 56 which includes the electrodes 54. Sheath 52 includes a wire extension 57 which includes the additional anode electrode 55 and the electrical contact 59. The curved distal end 56 wraps around the spinal cord or the nerves. This is another form of a fixing device. The stilet 61 can be inserted through passage 60 to maintain the distal end 56 linear until it is adjacent to the nerve or the spinal cord. The passage 60 may then be used for fluid delivery.

Different shape stilets may be used (bent tips for example) to improve steerability during positioning of the catheter, as illustrated in Figure 14.

Additional electrode 55 has the same length L1 along the sheath 52 or wire 57 as does the electrodes 54. The difference is that electrodes 54 are illustrated as 270° electrodes, whereas additional electrode 55 is a 180° electrode. This difference in circumferential dimension provides the difference in surface area. This provides the minimum 2 to 1 surface area as previously discussed. Obviously, the distance D1 between electrodes 54 is substantially less than the distance D2 between electrodes 54 and 55.

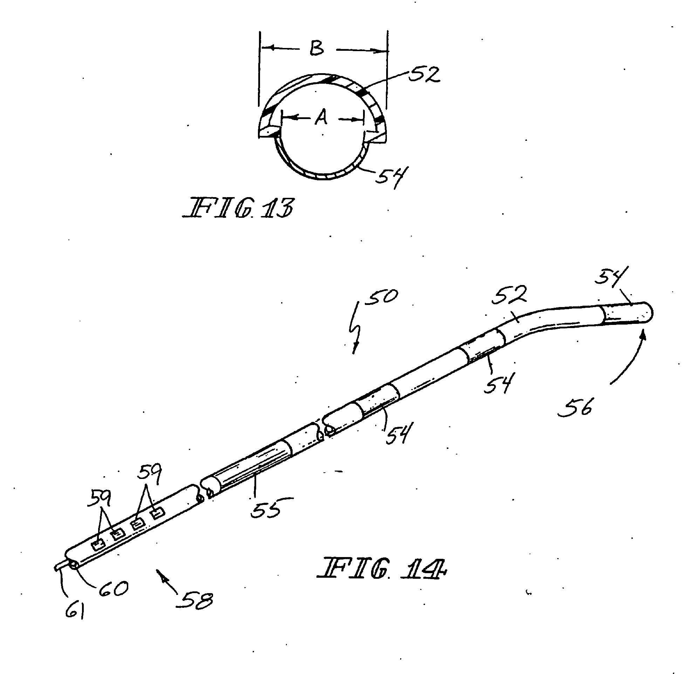

A modified 90° electrode is illustrated in cross-section in Figure 13. Electrode 54 has a smaller radius or diameter A than sheath's 52 radius or diameter B. For example, the diameter A may be 0.11 mm (0.0045 inches) and diameter B may be 1.7 mm (0.065 inches). The shape of the sheath and electrode allows introduction through a standard introducer needle. It still offers a reduced area electrode 54 and a stabilizing shape which may be used with or without tines or other fixation means.

Although the invention has been described for use with an implanted stimulator system (externally or internally powered), it should be noted that the same type of regime can be delivered by a non-implantable device. Applications for such non-implantable systems could include intra-operative testing of a stimulator system prior to implantation. An external stimulator system is connected to an implantable electrode for a period of trial stimulation prior to determining whether an implantation should be performed.

Although the present invention has been described and illustrated in detail, it is to be clearly understood that the same is by way of illustration and example only, and is not to be taken by way of limitation. The scope of the present invention is to be limited only by the terms of the appended claims.

## Claims

1. A catheter to lie along and stimulate tissue, comprising:
a sheath (52) having a distal end (56) and a proximal end (58),
at least one electrode (54) along the exterior of the distal end (56) of the sheath (52) to lie along tissue,
a passage (60) extending from an inlet at the proximal end (58) of the sheath (52) to an outlet (62) at the tip of the distal end (56) of the sheath (52), and
at least one fibre-optic channel (70, 72) in the passage (60) extending from a port at the proximal end (58) of the sheath (52) to a port at the distal end (56) of the sheath (52), the fibre-optic channel (70, 72) being adapted to provide photonic energy to the tissue and/or to function as a lens for a remote camera,
**characterized in that** said passage (60) has another outlet (64) adjacent to the tip of the distal end (56) and/or on the electrode (54).

2. The catheter of claim 1 wherein the port at the distal end (56) of the sheath (52) is at the tip of the distal end (56) and/or adjacent to the tip of the distal end and/or on at least one electrode (54).

## Patentansprüche

1. Katheter zum Anlegen an und Stimulieren von Gewebe, mit
einem Mantel (52), der ein distales Ende (56) und ein proximales Ende (58) aufweist,
mindestens einer Elektrode (54) längs dem Äußeren des distalen Endes (56) des Mantels (52) zum Anlegen an Gewebe,
einer von einem Einlaß am proximalen Ende (58) des Mantels (52) zu einem Auslaß (62) an der Spitze des distalen Endes (56) des Mantels (52) verlaufenden Durchführung (60) und
mindestens einem in der Durchführung (60) angeordneten Faseroptikkanal (70, 72), der von einer Öffnung am proximalen Ende (58) des Mantels (52) zu einer Öffnung am distalen Ende (56) des Mantels (52) verläuft und so ausgelegt ist, daß er dem Gewebe Photonenenergie zuführt und/oder als Linse für eine Fernkamera arbeitet,
**dadurch gekennzeichnet, daß** die Durchführung (60) einen weiteren Auslaß (64) nahe der Spitze des distalen Endes (56) und/oder an der Elektrode (54) aufweist.

2. Katheter nach Anspruch 1, wobei die Öffnung am distalen Ende (56) des Mantels (52) an der Spitze des distalen Endes (56) und/oder nahe der Spitze des distalen Endes und/oder an mindestens einer Elektrode (54) liegt.

## Revendications

1. Cathéter destiné à reposer le long d'un tissu et à stimuler celui-ci, comprenant :
une gaine (52) comportant une extrémité distale (56) et une extrémité proximale (58) ;
au moins une électrode (54) le long de l'extérieur de l'extrémité distale (56) de la gaine (52) destinée à reposer le long du tissu ; et
un passage (60) s'étendant d'une entrée à l'extrémité proximale (58) de la gaine (52) à une sortie (62) à la pointe de l'extrémité distale (56) de la gaine (52), et
au moins un canal de fibre optique (70, 72) dans le passage (60) s'étendant d'un orifice à l'extrémité proximale (58) de la gaine (52) à un orifice à l'extrémité distale (56) de la gaine (52), le canal de fibre optique (70, 72) étant adapté pour délivrer une énergie photonique au tissu et/ou pour jouer le rôle de l'objectif pour une caméra distante,
**caractérisé en ce que** ledit passage (60) comporte une autre sortie (64) au voisinage de la pointe de l'extrémité distale (56) et/ou sur l'électrode (54).

2. Cathéter selon la revendication 1, dans lequel l'orifice à l'extrémité distale (56) de la gaine (52) se trouve à la pointe de l'extrémité distale (56) et/ou au voisinage de la pointe de l'extrémité distale et/ou sur au moins une électrode (54).
